# EUROPÄISCHE PATENTSCHRIFT

(11) **EP 1 631 628 B1**
(45) Veröffentlichungstag und Bekanntmachung des Hinweises auf die Patenterteilung: **27.06.2007**
(21) Anmeldenummer: 03730094.4
(22) Anmeldetag: 22.05.2003
(51) Int. Cl.: C09C 3/06

(54) **ANORGANISCHE LICHTABSORBIERENDE MIKROPIGMENTE UND DEREN VERWENDUNG**
INORGANIC LIGHT-ABSORBING MICROPIGMENTS AND THE USE THEREOF
MICRO-PIGMENTS INORGANIQUES ABSORBANT LA LUMIERE ET LEUR UTILISATION

(43) Veröffentlichungstag der Anmeldung: 08.03.2006
(73) Patentinhaber: KEMIRA PIGMENTS OY, 28840 Pori (FI)
(72) Erfinder: DAHMS, Gerd, 47138 Duisburg (DE); SEIDEL, Holger, 47055 Duisburg (DE); ANTINLUOMA, Olli-Pekka, 28120 Pori (FI); MULTISILTA, Riku, 28130 Pori (FI)
(74) Vertreter: Peterreins, Frank
(86) Internationale Anmeldenummer: PCT/EP2003/005367
(87) Internationale Veröffentlichungsnummer: WO 2004/104111

(56) Entgegenhaltungen:
- EP-A- 0 753 546
- EP-A- 1 093 795
- US-A- 5 665 466

## Beschreibung

Die Erfindung betrifft die Verwendung von anorganischen lichtabsorbierenden Micropigmenten in Lichtschutzmitteln oder kosmetischen Emulsionen, geeignete anorganische lichtabsorbierende Pigmente oder Micropigmente und Verfahren zu deren Herstellung.

Seit längerer Zeit sind Lichtschutzmittel und kosmetische Emulsionen bekannt, die mikronisiertes Titandioxid als Micropigment enthalten. Neben organischen Lichtschutzfiltern dient das mikronisierte bzw. mikrofeine Titandioxid dazu, Licht zu absorbieren und zu reflektieren. Bei üblichen Anwendungen kommt es insbesondere auf einen UVA- und UVB-Schutz der Lichtschutzmittel an, die eine entsprechende UV-A- und UV-B-absorbierende Wirkung aufweisen sollen.

Aufgrund der zunehmenden UV-Exposition der menschlichen Haut steigt die Nachfrage an wirksameren Lichtschutzmitteln bzw. Sonnenschutzmitteln, die eine verbesserte UV-A und UV-B-Absorption bewirken. Die Sonnenschutzmittel enthalten UV-Absorber oder Lichtfilter, die die UV-Strahlung im Allgemeinen durch so genannte strahlungslose Desaktivierung in unschädliche Wärme umwandeln. Als organische Substanzen kommen hierbei in erster Linie Benzophenon-Derivate, Hydroxinaphthochinone, Phenylbenzoxazole und Phenylbenzimidazole, Digalloyltrioleat, Aminobenzoesäureester, Salicylsäureester, alicyclische Dienone, Zimtsäureester, Benzalazin usw. in Frage. Da mit organischen Lichtschutzfiltern häufig keine sehr hohen Licht- oder Sonnenschutzfaktoren erreicht werden, und diese sich häufig unter UV-Bestrahlung zersetzen oder nach dem Auftragen auf die Haut unerwünschte Penetrationseigenschaften zeigen, werden Licht- oder Sonnenschutzformulierungen üblicherweise auch anorganische lichtabsorbierende Pigmente, insbesondere anorganische lichtabsorbierende Micropigmente zugesetzt. Beispiele geeigneter anorganischer Pigmente bzw. Micropigmente sind Titandioxid, Ceroxid, Zirkonoxid. Diese Pigmente absorbieren und reflektieren die Strahlung. Hierbei wirken die Micropigmente jedoch häufig als Halbleiter, so dass Elektronen vom Valenz- in das Leitfähigkeitsband überführt werden. Durch den damit verbundenen Elektronenbedarf wirken diese lichtaktivierten Micropigmente als Oxidantien, die eine hautschädigende Wirkung entfalten können. Dies vermindert die vorteilhafte Wirkung üblicher anorganischer lichtabsorbierender Mikropigmente. Durch die Behandlung der Oberfläche (Calcinierung und/oder Hydrophobierung mit Alkylsilanen) werden die reaktiven Zentren so markiert, dass keine photochemischen Prozesse mehr ablaufen können. Die Maskierung gelingt aber meistens nur teilweise, so dass immer noch reaktive Zentren übrige bleiben.

Fernerhin ist bekannt, dass microfeines TiO₂ zusammen mit organischen Lichtschutzfiltern wie Octylmethoxycinnamat antagonistische Effekte zeigt. Dies schränkt den Einsatz von TiO₂ stark ein.

Weiterhin agglomerieren übliche Micropigmente häufig bereits bei der Formulierung des Sonnenschutzmittels, beispielsweise eines Sonnenöls, einer Sonnenmilch, einer Sonnencreme, eines Sonnengelees, einer Sonnenlotion, eines Sonnensprayöls oder einer Sonnensprayemulsion, oder nach der Anwendung auf die Haut. Die Bildung größerer Teilchen verringert jedoch deren Wirkung als Sonnenschutzmittel und führt zudem zu einem unerwünschten Weißeln auf der Haut.

US 5 665 466 betrifft ein Behandlungsverfahren für Titandioxidpigmente und die Verwendung dieser Pigmente in der Papierherstellung. Die Titandioxidpigmente werden zunächst mit Aluminiumphosphat und nachfolgend mit Aluminiumoxid beschichtet. Zudem kann eine Magnesiumoxidschicht zusätzlich aufgebracht werden. Die Teilchengröße liegt im Bereich von 200 bis 300 nm. Das Pigment wird als Mattierungsmittel in der Papierherstellung eingesetzt. Die Beschichtung des Titandioxidpigments erfolgt, um die physikalische Pigmentretention auf Papier zu erhöhen. Bei der Papierherstellung wird das Pigment mit mindestens einem Harz gemeinsam eingesetzt.

EP-A-0 753 546 betrifft Titandioxidpigmente mit einer Oberflächenbeschichtung aus Aluminiumphosphat. Die Pigmente werden zur Herstellung von Papierlaminaten eingesetzt und dienen zur Behebung des Problems der Grauverfärbung.

EP-A-1 093 795 betrifft kosmetische und dermatologische Lichtschutzformulierungen in Form einer O-W-Makroemulsion oder O-W-Microemulsion. Die Microemulsionen enthalten eine filmbildende Komponente auf Polyvinylpyrrolidon-Copolymergrundlage. Micropigmente werden erwähnt, und es wird angegeben, dass Titandioxidteilchen eine hydrophobe Oberflächenbeschichtung aufweisen können.

Aufgabe der vorliegenden Erfindung ist die Bereitstellung von anorganischen lichtabsorbierenden Pigmenten, insbesondere Micropigmenten, die die Nachteile der bekannten Pigmente bzw. Micropigmente vermeiden und insbesondere eine verminderte Oxidanswirkung zeigen.

Die Aufgabe wird erfindungsgemäß gelöst durch die Verwendung von anorganischen lichtabsorbierenden Micropigmenten, auf die Aluminiumphosphate aufgebracht sind, in Lichtschutzmitteln oder kosmetischen Emulsionen.

Die Micropigmente können dabei aus allen geeigneten anorganischen lichtabsorbierenden Micropigmenten ausgewählt sein. Vorzugsweise sind sie ausgewählt aus TiO₂, Ce₂O₃, ZrO₂, ZnO und Gemischen davon. Besonders bevorzugt wird Titandioxid (TiO₂) als Micropigment eingesetzt.

In den Micropigmenten beträgt die mittlere Teilchengröße vorzugsweise 5 bis 100 nm, besonders bevorzugt 10 bis 50 nm.

Die Aluminiumphosphate können aus allen geeigneten Aluminiumphosphaten ausgewählt sein. Man unterscheidet hierbei insbesondere Aluminiumorthophosphat (AlPO₄), Aluminiummetaphosphat (Al(PO₃)₃), Monoaluminumphosphat (Al(H₂PO₄)₃) sowie Aluminiumpolyphosphate, die aus Al(OH)₃ und H₃PO₄ entstehen. Erfindungsgemäß wird insbesondere Aluminiumorthophosphat, AlPO₄, auf die anorganischen lichtabsorbierenden Micropigmente aufgebracht. Für eine weitere Beschreibung der Aluminiumphosphate kann auf Römpp, Chemielexikon, 9. Auflage, Georg Thieme Verlag Stuttgart, Stichwort "Aluminiumphosphate" verwiesen werden.

Bei dem Titandioxid kann es sich um beliebiges geeignetes Titandioxid handeln, das insbesondere mikronisiert bzw. mikrofein ist. Das Titandioxid kann unbehandelt oder vorbehandelt (gecoated durch Calcinierung, chemische oder physikalische Adsorption von organischen Stoffen etc.) vorliegen.

Von Titandioxid sind drei Modifikationen bekannt, Anatas, Brookit und Rutil. Erfindungsgemäß können alle Modifikationen eingesetzt werden, bevorzugt sind jedoch Anatas, Rutil und Mischformen davon.

Titandioxid wird üblicherweise nach dem Sulfat- oder Chlorid-Verfahren hergestellt. Für eine Beschreibung geeigneter Titandioxide kann auf Römpp, Chemielexikon, 9. Auflage, Georg Thieme Verlag Stuttgart, Stichwort "Titandioxid" verwiesen werden. Eine weitere Beschreibung geeigneter Titandioxide findet sich in US 3,981,737, US 4,375,989, US 5,165,995.

Das Titandioxid kann in hydrophober Form vorliegen. Dies bedeutet, dass das Titandioxid oberflächlich wasserabweisend sein kann. Diese Oberflächenbehandlung kann darin bestehen, dass die Pigmente nach an sich bekannten Verfahren mit einer dünnen hydrophoben Schicht versehen werden. Beispielsweise kann das Titandioxid mit organischen Siliciumverbindungen wie Alkoxisilanen oberflächlich hydrophobiert werden. Derartige Pigmente sind beispielsweise in DE-A-33 14 742 beschrieben. Die hydrophobe Oberflächenmodifikation kann zusätzlich zur erfindungsgemäßen Modifikation durchgeführt werden.

Erfindungsgemäß wird das Titandioxid vorzugsweise mit 0,1 bis 25 Gew.-%, besonders bevorzugt 0,5 bis 15 Gew.-%, insbesondere 1 bis 10 Gew.-% an Aluminiumphosphaten behandelt, bezogen auf das fertige behandelte Titandioxid.

Behandlungsverfahren sind aus dem Stand der Technik bekannt und beispielsweise beschrieben in den vorstehend genannten Schriften. Die Herstellung kann durch Auffällen von Aluminiumphosphaten aus einer gelöste Aluminiumphosphate enthaltenden wässrigen Dispersion der anorganischen, lichtabsorbierenden Pigmente erfolgen. Üblicherweise wird durch Veränderung des pH-Wertes der Dispersion (z. B. TiO₂) auf die Pigmente abgeschieden. Die Abscheidung erfolgt zum Beispiel als hydratisierte Oxide.

Es wurde erfindungsgemäß gefunden, dass Titandioxid, auf das Aluminiumphosphate aufgebracht sind, eine signifikant verminderte Photoaktivität und damit eine verbesserte Stabilität zeigt. Dies zeigt sich insbesondere in Untersuchungen, die mit dem chromametrischen Verfahren der Tayca Corporation durchgeführt werden. In diesem Verfahren werden die Titandioxid-Micropigmente im Gewichtsverhältnis 1:1 mit Butylenglykol für drei Minuten vermischt. Sodann wird das Gemisch für eine Stunde dem Sonnenlicht ausgesetzt. Anschließend wird die Verfärbung des Butylenglykols mit Hilfe eines Minolta Chromameter Cr-200 untersucht. Je höher der Verfärbungsgrad und damit die Extinktionsänderung ist, um so größer ist die Photoaktiviät der Titandioxid-Pigmente.

Bei der Untersuchung mit UV-Licht wird beispielsweise die Titandioxid/Butylenglykol-Paste auf einen Glasträger aufgebracht, der wiederum auf einen weißen Karton gelegt wird. Sodann wird von oben mit UV-Licht bestrahlt. Für eine weitere Beschreibung des chromametrischen Verfahrens kann auf eine entsprechende Produktinformation von Tayca Co. verwiesen werden.

Ohne an eine Theorie gebunden zu sein, ist es möglich, dass die Aluminiumphosphate als elektrostatische Abstandhalter wirken. Dem Aluminium kommt dabei eine Kalzinierwirkung zu, während dem Phosphat eine Dispergierwirkung zukommt.

Zusätzlich zur Behandlung mit Aluminiumphosphaten können die anorganischen lichtabsorbierenden Micropigmente, insbesondere Titandioxid-Pigmente mit organischen Polymeren als sterischen Abstandhaltern beschichtet sein. Dabei kommen beliebige geeignete organische Polymere zum Einsatz, die als sterische Abstandhalter wirken. Besonders bevorzugt werden Polyvinylpyrrolidon (PVP) und dessen Copolymere eingesetzt. Entsprechende Copolymere sind beispielsweise in DE-A-199 23 672 und DE-A-199 50 089 beschrieben.

Sofern eine Beschichtung mit organischen Polymeren als sterischen Abstandhaltern durchgeführt wird, beträgt die Menge an organischem Polymer vorzugsweise 0,1 bis 10 Gew.-%, besonders bevorzugt 0,5 bis 3 Gew.-%, bezogen auf die beschichteten Micropigmente. Geeignete Beschichtungsverfahren sind dem Fachmann bekannt. Die Beschichtung kann beispielsweise durch Einbringen der Micropigmente in eine wässrige PVP-Lösung und nachfolgendes Trocknen durchgeführt werden.

Das organische Polymer kann ein beliebiges geeignetes Molekulargewicht aufweisen, sofern die Wirkung als sterischer Abstandhalter aufrecht erhalten bleibt.

Das erfindungsgemäße Micropigment zeigt eine gute Abstandshaltung, was sich in niedrigen Schüttdichten zeigt. Das Micropigment agglomeriert in den Anwendungen nicht beim Auftrag auf die Haut oder beim Sprühtrocknen. Damit liegen beispielsweise in Lichtschutzmitteln oder in den kosmetischen Emulsionen auch nach dem Auftrag auf die Haut fein verteilte und nicht agglomerierte Micropigmente vor. Gerade die Agglomerate beeinträchtigen sonst die Wirkung der Micropigmente als Lichtschutzmittel beträchtlich.

Die Lichtschutzmittel oder kosmetischen Emulsionen, die die erfindungsgemäßen Micropigmente enthalten, weisen ein gutes Hautgefühl auf aufgrund der geringen Primärpartikelgrößen. Insbesondere entsteht kein sandiges Gefühl beim Auftrag auf die Haut.

Damit sind die erfindungsgemäßen anorganischen lichtabsorbierenden Micropigmente in einer Vielzahl von Lichtschutzmitteln oder kosmetischen Emulsionen vorteilhaft einsetzbar. Die Erfindung betrifft auch Lichtschutzmittel oder kosmetische Emulsionen, die ein wie vorstehend definiertes anorganisches lichtabsorbierendes Micropigment enthalten. Der Gehalt an Micropigmenten in diesen Lichtschutzmitteln oder kosmetischen Emulsionen beträgt vorzugsweise 1 bis 40 Gew.-%, besonders bevorzugt 1 bis 15 Gew.-%, bezogen auf das gesamte Lichtschutzmittel oder die gesamte kosmetische Emulsion.

Lichtschutzmittel können dabei beispielsweise als Sonnen-Öl, -milch(Emulsion), -creme, -gelee, -lotion, -sprayöl oder -sprayemulsion formuliert werden. Die Lichtschutzmittel oder kosmetischen Emulsionen können beispielsweise in Forme einer OW-, WO-, PO-, POW-Emulsion oder anderen multiplen Emulsion vorliegen.

Die Lichtschutzmittel oder kosmetischen Emulsionen können weitere Komponenten enthalten wie kosmetisch oder pharmazeutisch wirksame Substanzen, organische wasserlösliche Lichtschutzfilter, andere, hydrophil beschichtete Mikropigmente, Elektrolyte, Glycerin, Polyethylenglykol, Propylenglykol, Bariumsulfat, Alkohole, Wachse, Metallseifen wie Magnesiumstearat, Vaseline oder andere Inhaltsstoffe.

Beispielsweise können weiterhin Parfums, Parfumöle oder -aromen zugesetzt werden. Geeignete Zusatzstoffe sind im Folgenden beispielhaft aufgeführt.

Geeignet sind kosmetische Wirkstoffe, die insbesondere oxidations- oder hydrolyseempfindlich sind wie beispielsweise Polyphenole. Hier seien genannt Catechine (wie Epicatechin, Epicatechin-3gallat, Epigallocatechin, Epigallocatechin-3-gallat), Flavonoide (wie Luteolin, Apigenin, Rutin, Quercitin, Fisetin, Kaempherol, Rhametin), Isoflavone (wie Genistein, Daidzein, Glycitein, Prunetin), Cumarine (wie Daphnetin, Umbelliferon), Emodin, Resveratrol, Oregonin.

Geeignet sind Vitamine wie Retinol, Tocopherol, Ascorbinsäure, Riboflavin, Pyridoxin.

Geeignet sind ferner Gesamtextrakte aus Pflanzen, die u. a. obige Moleküle oder Molekülklassen enthalten.

Bei den Wirkstoffen handelt es sich gemäß einer Ausführungsform der Erfindung um Lichtschutzfilter. Diese können als organische Lichtschutzfilter bei Raumtemperatur (25 °C) in flüssiger oder fester Form vorliegen. Geeignete Lichtschutzfilter (UV-Filter) sind beispielsweise Verbindungen auf Basis von Benzophenon, Diphenylcyanacrylat oder p-Aminobenzoesäure. Konkrete Beispiele sind (INCI- oder CTFA-Bezeichnungen) Benzophenone-3, Benzophenone-4, Benzophenone-2, Benzophenone-6, Benzophenone-9, Benzophenone-1, Benzophenone-11, Etocrylene, Octodrylene, PEG-25 PABA, Phenylbenzimidazole Sulfonic Acid, Ethylhexyl Methoxycinnamate, Ethylhexyl Dimethyl PABA, 4-Methylbenzylidene Camphor, Butyl Methoxydibenzoylmethane, Ethylhexyl Salicylate, Homosalate sowie Methylene-bis-benzotriazolyl Tetramethylbutylphenol (2,2'-Methylen-bis-{6-(2H-benzoetriazol-2-yl)-4-(1,1,3,3-tetramethylbutyl)-phenol}, 2-Hydroxy-4-methoxybenzophenon-5-sulfonsäure und 2,4,6-Trianilino-p-(carbo-2'-ethylhexyl-1'-oxi)-1,3,5-triazin.

Weitere organische Lichtschutzfilter sind Octyltriazone, Avobenzone, Octylmethoxycinnamate, Octylsalicylate, Benzotriazole und Triazine.

Gemäß einer weiteren Ausführungsform der Erfindung werden als Wirkstoffe Antischuppen-Wirkstoffe eingesetzt, wie sie üblicherweise in kosmetischen oder pharmazeutischen Formulierungen vorliegen. Ein Beispiel hierfür ist Piroctone Olamine (1-Hydroxy-4-methyl-6-(2,4,4-dimethylpentyl)-2(1H)-pyridone; vorzugsweise in Kombination mit 2-Aminoethanol (1:1)). Weitere geeignete Mittel zur Behandlung von Hautschuppen sind dem Fachmann bekannt.

Die beschriebene P/O-Emulsion kann auch in Wasser oder eine Wasser-in-Öl-Emulsion emulgiert werden. Dabei resultiert eine Polyol-in-Öl-in-Wasser-Emulsion (P/O/W-Emulsion), die mindestens eine beschriebene Emulsion und zusätzlich mindestens eine wässrige Phase enthält. Derartige multiple Emulsionen können im Aufbau den in DE-A-43 41 113 beschriebenen Emulsionen entsprechen.

Beim Einbringen der erfindungsgemäßen P/O-Emulsion in Wasser oder wässrige Systeme kann das Gewichtsverhältnis der einzelnen Phasen in weiten Bereichen variiert werden. Vorzugsweise beträgt in der letztendlich erhaltenen P/O/W-Emulsion der Gewichtsanteil der P/O-Emulsion 0,01 bis 80 Gew.-%, besonders bevorzugt 0,1 bis 70 Gew.-%, insbesondere 1 bis 30 Gew.-%, bezogen auf die gesamte P/O/W-Emulsion.

Beim Einbringen der erfindungsgemäßen P/O-Emulsion in eine O/W-Emulsion beträgt der Anteil der P/O-Emulsion vorzugsweise 0,01 bis 60 Gew.-%, besonders bevorzugt 0,1 bis 40 Gew.-%, insbesondere 1 bis 30 Gew.-%, bezogen auf die letztendlich erhaltene P/O/W-Emulsion. In der O/W-Emulsion, die hierzu verwendet wird, beträgt der Ölanteil vorzugsweise 1 bis 80 Gew.-%, besonders bevorzugt 1 bis 30 Gew.-%, bezogen auf die eingesetzte O/W-Emulsion. Anstelle einer P/O-Emulsion kann auch eine W/O-Emulsion eingebracht werden, was zu einer W/O/W-Emulsion führt. Die einzelnen Phasen der Emulsionen können noch übliche für die einzelnen Phasen bekannte Inhaltsstoffe aufweisen. Beispielsweise können die einzelnen Phasen weitere in diesen Phasen lösliche pharmazeutische oder kosmetische Wirkstoffe enthalten. Die wässrige Phase kann beispielsweise organische lösliche Lichtschutzfilter, hydrophil gecoatetes Micropigment, Elektrolyte, Alkohole usw. enthalten. Einzelne oder alle der Phasen können zudem Feststoffe enthalten, die vorzugsweise ausgewählt sind aus Pigmenten oder Micropigmenten, Mikrosphären, Silikagel und ähnlichen Stoffen. Die Ölphase kann beispielsweise organisch modifizierte Tonmineralien, hydrophob gecoatete (Micro)Pigmente, organische öllösliche Lichtschutzfilter, öllösliche kosmetische Wirkstoffe, Wachse, Metallseifen wie Magnesiumstearat, Vaseline oder Gemische davon enthalten. Als (Micro)Pigmente können Titandioxid, Zinkoxid und Bariumsulfat sowie Wollastonit, Kaolin, Talk, Al₂O₃, Bismutoxidchlorid, micronisiertes Polyethylen, Glimmer, Ultramarin, Eosinfarben, Azofarbstoffe, genannt werden. Insbesondere Titandioxid oder Zinkoxid sind in der Kosmetik als Lichtschutzfilter gebräuchlich und lassen sich mittels der erfindungsgemäßen Emulsionen besonders glatt und gleichmäßig auf die Haut auftragen. Mikrosphären oder Silicagel können als Träger für Wirkstoffe eingesetzt werden, und Wachse können beispielsweise als Grundlage für Polituren verwendet werden.

Zur Herstellung der erfindungsgemäßen kosmetischen Emulsionen werden in der Regel Emulgatoren verwendet. Beispiele geeigneter Emulgatoren sind Glycerinester, Polyglycerinester, Sorbitanester, Sorbitolester, Fettalkohole, Propylenglykolester, Alkylglucosidester, Zuckerester, Lecithin, Silikoncopolymere, Wollwachs und ihre Mischungen und Derivate. Glycerinester, Polyglycerinester, Alkoxylate und Fettalkohole sowie Isoalkohole können sich beispielsweise ableiten von Rhizinusfettsäure, 12-Hydroxystearinsäure, Isostearinsäure, Ölsäure, Linolsäure, Linolensäure, Stearinsäure, Myrestinsäure, Maurinsäure und Caprinsäure. Neben den genannten Estern können auch Succinate, Amide oder Ethanolamide der Fettsäuren vorliegen. Als Fettsäurealkoxylate kommen insbesondere die Ethoxylate, Propoxylate oder gemischten Ethoxylate/Propoxylate in Betracht. Ferner können Emulgatoren eingesetzt werden, die Lamelarstrukturen ausbilden. Beispiele derartiger Emulgatoren sind die physiologischen Gallensalze wie Natriumcheolat, Natriumdehydrocheolat, Natriumdeoxycheolat, Natriumglycochealat, Natriumtaurochealat. Tierische und pflanzliche Phospholipide wie Lecithine mit Ihren hydrierten Formen sowie Polypeptide wie Gelatine mit ihren modifizierten Formen können ebenso verwendet werden.

Als synthetische grenzflächenaktive Substanzen eignen sich die Salze der Sulfobemsteinsäureester, Polyoxiethylensäurebethanester, Säurebethanester und Sorbitanether, Polyoxiethylenfettalkoholether, Polyoxiethylenstearinsäureester sowie entsprechende Mischungskondensate von Polyoxiethylen-methpolyoxipropylenethern, ethoxylierte gesättigte Glyceride, partielle Fettsäure-Glyceride und Polyglycide. Beispiele geeigneter Tenside sind Biobase^{®} ED und Ceralution.

Als wässrige Phase können Wasser, wässrige Lösungen oder Mischungen von Wasser mit wassermischbaren Flüssigkeiten wie Glycerin oder Polyethylenglykol eingesetzt werden. Ferner können in der wässrigen Phase Elektrolyte wie Natriumchlorid enthalten sein. Falls gewünscht, können ferner viskositätserhöhende Stoffe oder Ladungsträger eingesetzt werden, wie sie in der EP-B-0605 497 beschrieben sind.

Die Wasserphase kann darüber hinaus Propylenglykol, Ethylenglykol und ähnliche Verbindungen sowie Derivate davon enthalten.

Die Verwendung von üblichen Hilfs- und Zusatzstoffen in den Emulsionen ist dem Fachmann bekannt.

Die Erfindung betrifft nicht nur den Einsatz der beschriebenen anorganischen lichtabsorbierenden Micropigmente, sondern auch entsprechende anorganische lichtabsorbierende Micropigmente mit einer mittleren Teilchengröße von 5 bis 100 nm, auf die Aluminiumphosphate aufgebracht sind. Die erfindungsgemäße Beschichtung der Micropigmente weist auch in anderen Anwendungsgebieten Vorteile auf, in denen größere Pigmente anstelle von Micropigmenten eingesetzt werden. Beispielsweise bei eingefärbten Kunststoffen und Papier, die jeweils anorganische lichtabsorbierende Pigmente enthalten, tritt ebenfalls das Zersetzungsproblem unter Einfluss von Licht auf. Auch in diesen Anwendungen führt der Einsatz der erfindungsgemäß beschichteten anorganischen lichtabsorbierenden Pigmente zu Vorteilen. Bei diesen Anwendungen genügt es, auf die Pigmente Aluminiumphosphate aufzubringen. Das Aufbringen von organischen Polymeren als sterischen Abstandhaltern ist häufig nicht notwendig, da bereits größere Pigmentteilchen vorliegen.

Die Erfindung betrifft auch die Kombination bestehend aus den anorganischen lichtabsorbierenden Micropigmenten mit einer mittleren Teilchengröße von 5 bis 100 nm, auf die AlPO₄ aufgebracht sind, und organischen Lichtschutzfiltern insbesondere Octylmethoxycinnamate. Insbesondere die Kombination aus microfeinem TiO₂ mit aufgezogenem AlPO₄ zeigt im Gegensatz zu konventionellen TiO₂ Qualitäten synergistische Effekte. Die anorganischen Pigmente und die organischen Lichtschutzfilter werden vorzugsweise in einem Gewichtsverhältnis von 1 : 10 bis 10 : 1 eingesetzt

Weitere Anwendungen für die erfindungsgemäßen anorganischen lichtabsorbierenden Pigmente, insbesondere Mikropigmente, sind dem Fachmann bekannt.

Die Erfindung wird durch die nachstehenden Beispiele näher erläutert.

### Beispiele

### Verfahrenschritte zur Herstellung von PO4/PVP-oberflächenmodifiziertem Titandioxid

Zu Oberflächenmodifizierung wird reines, nicht-dotiertes Titandioxid eingesetzt, welches nach der Herstellung zunächst calciniert, vermahlen und daraufhin filtriert wurde. Die Mikropigment-Filterfraktion wird in Wasser aufgeschwemmt und im sauren Medium mit Phosphat versetzt. Durch Einstellung des pH-Wertes wird eine Phosphatfällung durchgeführt, der sich ein weiterer Trocknungsprozess anschließt. Das phosphatierte Zwischenprodukt wird nun in einem weiteren Verfahrensschritt unter Rühren mit einer wässrigen Polyvinylpyrollidon-Lösung versetzt. Der Filterrückstand dieser Suspension wird getrocknet und erneut filtriert.

Zunächst wurde die Photostabilität der erfindungsgemäßen Micropigmente gegenüber bekannten Micro- und Makropigmenten untersucht. Hierzu wurde das vorstehend beschriebene chromametrische Verfahren von Tayca Corporation angewendet.

### Beispiel 1

Als erfindungsgemäßes Micropigment wurde ein Titandioxid-Micropigment mit einer Primärpartikelgröße im Bereich von 16 bis 24 nm eingesetzt. Die Titandioxid-Pigmente waren mit AlPO₄- beschichtet. Der Gehalt an AlPO₄- betrug etwa 7 Gew.-%, bestimmt als Al₂O₃ und 1 bis 1,5 Gew. % bestimmt als P₂O₅. Die Partikel waren auch PVP-beschichtet mit 2,0 Gew.-%, bezogen auf TiO2.

Zu Vergleichszwecken wurden unterschiedliche handelsübliche Titandioxid-Pigmente eingesetzt.

Die Pigmente wurden im Massenverhältnis 1:2 mit Butylenglykol für drei Minuten vermischt. Die erhaltene Paste wurde für eine Stunde einer UV-Lichtquelle ausgesetzt. Der Abstand zwischen der Probe und der UV-Lichtquelle betrug 30 cm.

Vor und nach dem Bestrahlen wurde die Extinktion mit einem Minolta Chromameter CR-300 bestimmt. Sodann wurde der Grad an Verfärbung bestimmt, wobei ein großer Zahlenwert eine starke Verfärbung angibt. Die Verfärbung ist ein direktes Maß für die Photoaktivität der Titandioxid-Partikel. Photostabile Titandioxid-Partikel führen zu einer geringen Verfärbung in der TitandioxidButylenglykol-Paste.

Die Ergebnisse sind in der nachstehenden Tabelle zusammengefasst:

| Pigmentart | Hersteller | Verfärbungsgrad |
|---|---|---|
| erfindungsgemäß | **Kemira** | **2,90** |
| UV-Titan M262 | Kemira | 8,26 |
| UV-Titan M212 | Kemira | 9,05 |
| Cardre TiO2-Si2 | Cardre | 14,11 |
| Cardre TiO2-AS | Cardre | 15,27 |
| Eusolex T 2000 | Sachtleben | 28,90 |
| Tayca MT100T | Tayca Corp. | 48,77 |

### Beispiel 2:

Unterschiedliche Titandioxid-Mikropigmente und aus dem Stand der Technik bekannte Mikropigmente und Makropigmente wurden in Alkylbenzoat dispergiert. Als erfindungsgemäßes Micropigment wurde das in Beispiel 1 beschriebene Micropigment eingesetzt. Es wurden jeweils 10 Gew.-% Titandioxid in Alkylbenzoat dispergiert, das ein Gew.-% Ascorbylpalmitat enthielt. Die Gewichtsanteile beziehen sich auf die fertige Zusammensetzung. Als Referenz wurde eine Mischung aus 10 % Titandioxid, dispergiert in Alkylbenzoat, eingesetzt. Es wurde jeweils für eine Minute vermischt.

Die Messung mit dem Minolta Chromameter CR-300 und die Bestrahlung wurde wie im Beispiel 1 beschrieben durchgeführt. Die Ergebnisse sind in der nachfolgenden Tabelle zusammengefasst:

| Pigmentqualität | Hersteller | Verfärbungsgrad |
|---|---|---|
| erfindungsgemäß | Kemira | **0,71** |
| UV-Titan M262 | Kemira | 1,10 |
| UV-Titan M212 | Keinira | 1,28 |
| Cardre TiO2-AS | Cardre | 4,00 |
| Eusolx T 2000 | Sachtleben | 4,78 |
| Tayca MT100T | Tayca Cop. | 20,40 |

### Beispiel 3:

Erfindungsgemäße Micropigmente mit unterschiedlichen AlPO₄ Mengen wurde bezüglich ihrer Photostabilität untersucht und deren Verfärbungsgrad wurde über die Zeit mit dem Chromameter bestimmt. Folgende Pigmente kamen zum Einsatz

| | | | | |
|---|---|---|---|---|
| **Zeit [h]:** | 0,0166 | 1 | 18 | 120 |
| | | | | |
| **SAMPLE 7/1** | **0,71** | **1,65** | **1,91** | **3,09** |
| **SAMPLE 7/2** | **0,95** | **1,88** | **2,8** | **3,31** |
| **SAMPLE 7/6** | **2,91** | **5,63** | **7,53** | **8,07** |
| **SAMPLE7/7** | **1,50** | **2,45** | **4,06** | **5,4** |

| | | | | |
|---|---|---|---|---|
| Sample 7/1 enthielt 1,2 % P205, Sample 7/2 2,4 % P205. Beide Proben enthielten 2 % PVP. | | | | |

### Beispiel 4:

Im folgenden Beispiel wird der synergistische Effekt von microfeinem TiO₂ auf das AlPO₄ in Kombination mit PVP aufgezogen wurde, dargestellt (SPF = Sun Protection Factor).

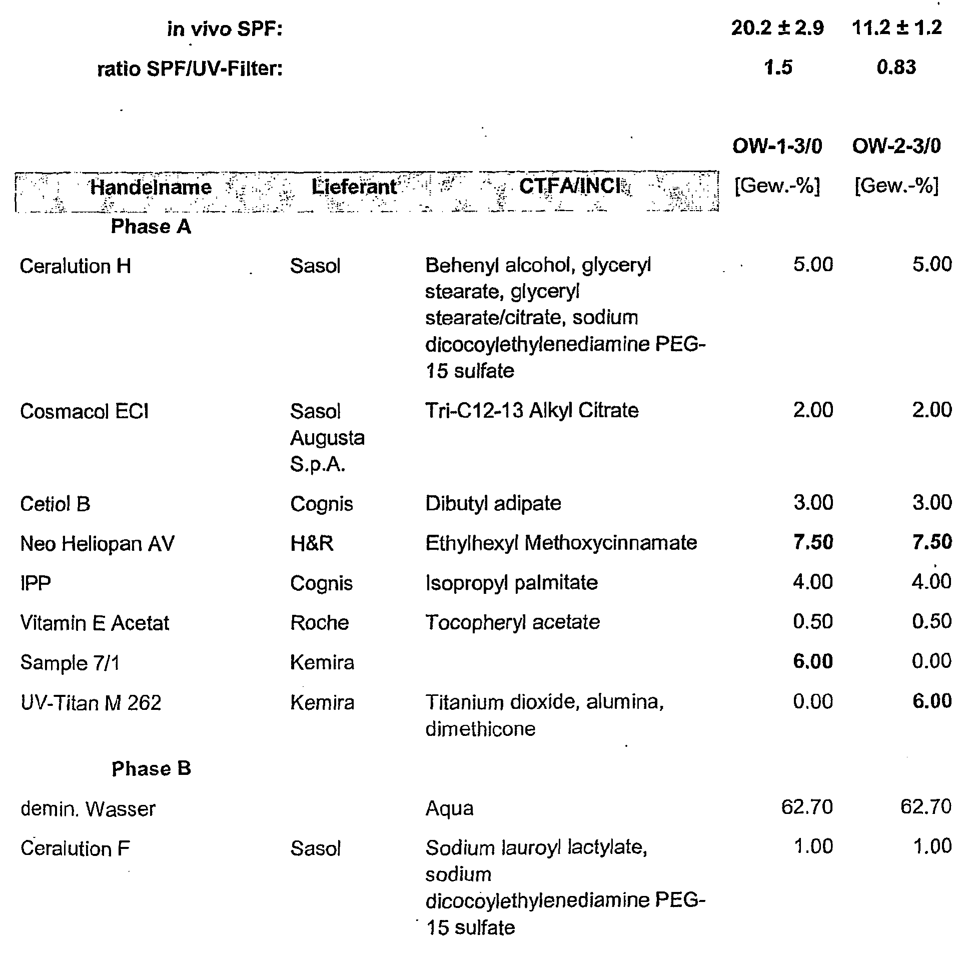

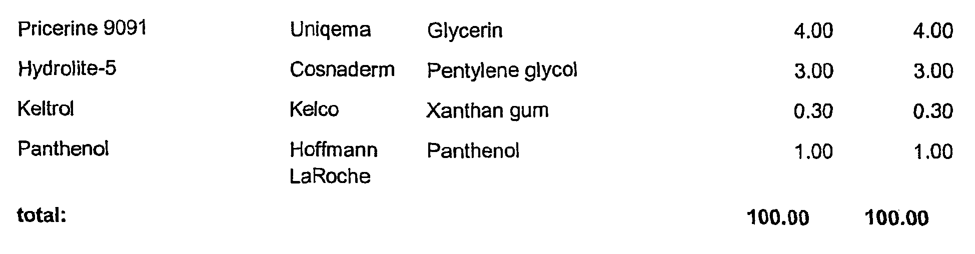

### Herstellung

Zur Herstellung der Sonnenschutzformulierung wurden die Phasen A und B getrennt auf 60 bis 70 °C erwärmt. Sodann wurde Phase A zu B gegeben, und es wurde für zwei Stunden homogenisiert. Sodann wurde auf 40 °C abgekühlt und für weitere zwei Minuten Homogenisiert.

## Patentansprüche

1. Verwendung von anorganischen lichtabsorbierenden Micropigmenten auf die Aluminiumphosphate aufgebracht sind, in Lichtschutzmitteln oder kosmetischen Emulsionen
mit Ausnahme von mit Phosphaten umhülltes Metalloxid mit einer mittleren Länge der Primärteilchen im Bereich von 50 bis 90 nm und einer mittleren Breite der Primärteilchen im Bereich von 5 bis 20 nm.

2. Verwendung nach Anspruch 1, **dadurch gekennzeichnet, dass** das anorganische lichtabsorbierende Micropigment zusätzlich mit organischen Polymeren als sterischen Abstandhaltern beschichtet ist.

3. Verwendung von anorganischen lichtabsorbierenden Micropigmenten, auf die Aluminiumphosphate aufgebracht sind und das anorganische lichtabsorbierende Micropigment zusätzlich mit organischen Polymeren als sterischen Abstandhaltern beschichtet ist, in Lichtschutzmitteln oder kosmetischen Emulsionen.

4. Verwendung nach einem der Ansprüche 1 bis 3, **dadurch gekennzeichnet, dass** das anorganische lichtabsorbierende Micropigment ausgewählt ist aus TiO₂, Ce₂O₃, ZrO₂, ZnO und Gemischen davon.

5. Verwendung nach einem der Ansprüche 1 bis 4, **dadurch gekennzeichnet, dass** die mittlere Teilchengröße im anorganischen lichtabsorbierenden Micropigment 5 bis 100 nm beträgt.

6. Verwendung nach einem der Ansprüche 1 bis 5, **dadurch gekennzeichnet, dass** die Lichtschutzmittel oder kosmetischen Emulsionen in Form einer OW-, WO-, PO-. POW-Emulsion oder anderen multiplen Emulsion vorliegt.

7. Lichtschutzmittel oder kosmetische Emulsion, enthaltend ein anorganisches lichtabsorbierendes Micropigment, wie es in einem der Ansprüche 1 bis 5 definiert ist, und gegebenenfalls organische Lichtschutzfilter.

8. Lichtschutzmittel oder kosmetische Emulsion nach Anspruch 7, **dadurch gekennzeichnet, dass** sie in Form einer OW-, WO-, PO-. POW-Emulsion oder anderen multiplen Emulsion vorliegt.

9. Anorganisches lichtabsorbierendes Micropigment, auf das Aluminiumphosphate aufgebracht sind,
ausgenommen mit Phosphaten umhülltes Metalloxid mit einer mittleren Länge der Primärteilchen im Bereich von 50 bis 90 nm und einer mittleren Breite der Primärteilchen im Bereich von 5 bis 20 nm,
**dadurch gekennzeichnet, dass** die mittlere Teilchengröße im anorganischen lichtabsorbierenden Micropigment 5 bis 100 nm beträgt.

10. Micropigment nach Anspruch 9, **dadurch gekennzeichnet, dass** es zusätzlich mit organischen Polymeren als sterischen Abstandhaltern beschichtet ist.

11. Anorganisches lichtabsorbierendes Micropigment nach Anspruch 9, auf das Aluminiumphosphate aufgebracht sind, und das zusätzlich mit organischen Polymeren als sterischen Abstandhaltern beschichtet ist, **dadurch gekennzeichnet, dass** die mittlere Teilchengröße im anorganischen lichtabsorbierenden Micropigment 5 bis 100 nm beträgt.

12. Micropigment nach einem der Ansprüche 9 bis 11, **dadurch gekennzeichnet, dass** die mittlere Teilchengröße 10 bis 50 nm beträgt.

13. Verfahren zur Herstellung von anorganischen lichtabsorbierenden Micropigmenten nach einem der Ansprüche 9 bis 12 durch Auffällen von Aluminiumphosphaten aus einer gelöste Aluminiumphosphate enthaltenden wässrigen Dispersion der anorganischen lichtabsorbierenden Micropigmente.

## Claims

1. Use of inorganic light-absorbing micropigments to which aluminium phosphates are applied in sunscreens or cosmetics emulsions
except for metal oxide being coated with phosphates, wherein the mean length of the primary particles is in the range of 50 to 90 nm and the mean width of the primary particles is in the range of 5 to 20 nm.

2. Use according to claim 1, **characterized in that** the inorganic light-absorbing micropigment is additionally coated with organic polymers as steric spacers.

3. Use of inorganic light-absorbing micropigments to which aluminium phosphates are applied and the inorganic light-absorbing micropigment is additionally coated with organic polymers as steric spacers in sunscreens or cosmetics emulsions.

4. Use according to one of claims 1 to 3, **characterized in that** the inorganic light-absorbing micropigment is selected from TiO₂, Ce₂O₃, ZrO₂, ZnO and mixtures thereof.

5. Use according to one of claims 1 to 4, **characterized in that** the mean particle size in the inorganic light-absorbing micropigment is from 5 to 100 nm.

6. Use according to one of claims 1 to 5, **characterized in that** the sunscreens or cosmetics emulsions are present in the form of an OW, WO, PO, POW emulsion or other multiple emulsion.

7. Sunscreen or cosmetics emulsion comprising an inorganic light-absorbing micropigment as defined in one of claims 1 to 5, with or without organic light-protection filters.

8. Sunscreen or cosmetics emulsion according to claim 7, **characterized in that** it is present in the form of an OW, WO, PO, POW emulsion or other multiple emulsions.

9. Inorganic light-absorbing micropigment to which aluminium phosphates are applied
except for metal oxide being coated with phosphates, wherein the mean length of the primary particles is in the range of 50 to 90 nm, and the mean width of the primary particles is in the range of 5 to 20 nm,
**characterized in that** the mean particle size in the inorganic light-absorbing micropigment is from 5 to 100 nm.

10. Micropigment according to claim 9, **characterized in that** it is additionally coated with organic polymers as steric spacers.

11. Inorganic light-absorbing micropigment according to claim 9 to which aluminium phosphates are applied and which is additionally coated with organic polymers as steric spacers, **characterized in that** the mean particle size in the inorganic light-absorbing micropigment is from 5 to 100 nm.

12. Micropigment according to one of claims 9 to 11, **characterized in that** the mean particle size is from 10 to 50 nm.

13. Process for producing inorganic light-absorbing micropigments according to one of claims 9 to 12 by precipitating aluminium phosphates from an aqueous dispersion of the inorganic light-absorbing micropigments which contains dissolved aluminium phosphates.

## Revendications

1. Utilisation de micropigments inorganiques absorbant la lumière, sur lesquels sont appliqués des phosphates d'aluminium, dans des agents de protection contre la lumière ou des émulsions cosmétiques, à l'exception d'un oxyde métallique, enveloppé de phosphates, ayant une longueur moyenne des particules primaires comprise dans la plage de 50 à 90 nm et une largeur moyenne des particules primaires comprise dans la plage de 5 à 20 nm.

2. Utilisation selon la revendication 1, **caractérisée en ce que** le micropigment inorganique absorbant la lumière est en outre revêtu de polymères organiques, servant d'éléments écarteurs stériques.

3. Utilisation de micropigments inorganiques absorbant la lumière, sur lesquels sont appliqués des phosphates d'aluminium, et le micropigment inorganique absorbant la lumière est en outre revêtu de polymère organiques en tant qu'éléments écarteurs stériques, dans des agents de protection contre la lumière ou des émulsions cosmétiques.

4. Utilisation selon l'une des revendications 1 à 3, **caractérisée en ce que** le micropigment inorganique absorbant la lumière est choisi parmi TiO₂, Ce₂O₃, ZrO₂, ZnO et leurs mélanges.

5. Utilisation selon l'une des revendications 1 à 4, **caractérisée en ce que** la granulométrie moyenne du micropigment inorganique absorbant la lumière est de 5 à 100 nm.

6. Utilisation selon l'une des revendications 1 à 5, **caractérisée en ce que** les agents de protection contre la lumière ou les émulsions cosmétiques se présentent sous forme d'une émulsion huile-dans-l'eau, eau-dans-l'huile, polyol-dans-l'huile, polyol-dans-l'huile-dans-l'eau, ou d'autres émulsions multiples.

7. Agent de protection contre la lumière ou émulsion cosmétique contenant un micropigment inorganique absorbant la lumière, tel que défini dans l'une des revendications 1 à 5, et éventuellement des filtres organiques de protection contre la lumière.

8. Agent de protection contre la lumière ou émulsion cosmétique selon la revendication 7, **caractérisé en ce qu'**il se présente sous forme d'une émulsion huile-dans-l'eau, eau-dans-l'huile, polyol-dans-l'huile, polyol-dans-l'huile-dans-l'eau, ou d'une autre émulsion multiple.

9. Micropigment inorganique absorbant la lumière, sur lequel sont appliqués des phosphates d'aluminium, à l'exception d'un oxyde métallique enveloppé de phosphates, ayant une longueur moyenne des particules primaires comprise dans la plage de 50 à 90 nm et une largeur moyenne des particules primaires comprise dans la plage de 50 à 20 nm, **caractérisé en ce que** la granulométrie moyenne du micropigment inorganique absorbant la lumière est de 5 à 100 nm.

10. Micropigment selon la revendication 9, **caractérisé en ce qu'**il est en outre enrobé de polymères organiques servant d'éléments écarteurs stériques.

11. Micropigment inorganique absorbant la lumière selon la revendication 9, sur lequel sont appliqués des phosphates d'aluminium et qui en outre est revêtu de polymères inorganiques servant d'éléments écarteurs stériques, **caractérisé en ce que** la granulométrie moyenne du micropigment inorganique absorbant la lumière est de 5 à 100 nm.

12. Micropigment selon l'une des revendications 9 à 11, **caractérisé en ce que** la granulométrie moyenne est de 10 à 50 nm.

13. Procédé de fabrication de micropigmènts inorganiques absorbant la lumière selon l'une des revendications 9 à 12, par précipitation de phosphates d'aluminium sur une dispersion aqueuse, contenant des phosphates d'aluminium dissous, des micropigments inorganiques absorbant la lumière.
